# EUROPÄISCHE PATENTSCHRIFT

(11) **EP 2 812 054 B1**
(45) Veröffentlichungstag und Bekanntmachung des Hinweises auf die Patenterteilung: **22.06.2016**
(21) Anmeldenummer: 13704002.8
(22) Anmeldetag: 06.02.2013
(51) Int. Cl.: A61M 5/315, A61M 5/24

(54) **INJEKTIONSGERÄT**
INJECTION DEVICE
DISPOSITIF D'INJECTION

(30) Priorität: 10.02.2012 DE 202012001410 U
(43) Veröffentlichungstag der Anmeldung: 17.12.2014
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2013/000358
(87) Internationale Veröffentlichungsnummer: WO 2013/117331

(56) Entgegenhaltungen:
- EP-B1- 1 610 848

## Beschreibung

Die Erfindung betrifft ein Injektionsgerät der im Oberbegriff des Anspruchs 1 angegebenen Gattung.

Aus der EP 1 610 848 B2 ist ein Injektionsgerät bekannt, bei dem eine Drehbewegung des Bedienelements über zwei Gewindeverbindungen in eine axiale Bewegung eines Zustellteil in distaler Richtung umgewandelt wird. Zum Auspressen der Injektion-flüssigkeit aus dem Behälter wird das Zustellteil in axialer Richtung bewegt und wirkt so auf einen Dosierkolben, der die Injektionsflüssigkeit aus dem Behälter auspresst.

Bei dem in der EP 1 610 848 B2 gezeigten Injektionsgerät können unterschiedlichste Mengen an Injektionsflüssigkeit eingestellt werden. Die möglichen einzustellenden Mengen werden über eine Rasteinrichtung vorgegeben, die so ausgelegt ist, dass das Bedienelement nur in Positionen gestellt werden kann, die zulässigen eingestellten Mengen an Injektionsflüssigkeit entsprechen. Von Positionen des Bedienelements, die unzulässigen Mengen an Injektionsflüssigkeit entsprechen, springt das Bedienelement in die nächstliegende zulässige Position.

Damit der Bedienknopf aus einer Zwischenposition selbsttätig und sicher in eine Rastposition springt, muss die Rastung hinreichend stark sein, und die radialen Rastpositionen müssen hinreichend nahe beieinander liegen. Die Stärke der Rastung beeinflusst jedoch das Drehmoment, das der Anwender aufwenden muss, um den Bedienknopf zu drehen und die Dosis einzustellen. Der konstruktiv mögliche Abstand der Rastpositionen ist weitgehend vorgegeben und kann nur in engen Grenzen auf den Anwendungsfall angepasst werden.

Der Erfindung liegt die Aufgabe zugrunde, ein Injektionsgerät der gattungsgemäßen Art zu schaffen, bei dem nur vorgegebene Mengen von Injektionsflüssigkeit aus dem Behälter ausgepresst werden können und das eine gute Anpassung auf den gewünschten Anwendungsfall erlaubt.

Diese Aufgabe wird durch ein Injektionsgerät mit den Merkmalen des Anspruchs 1 gelöst.

Das Injektionsgerät besitzt ein Zustellteil, das beim Einstellen der auszupressenden Menge an Injektionsflüssigkeit in distaler Richtung bewegt und gleichzeitig um die Längsmittelachse gedreht wird. Beim Auspressen der Injektionsflüssigkeit bewegt sich das Zustellteil in Richtung der Längsmittelachse in proximaler Richtung, dreht sich gegenüber dem Gehäuse jedoch nicht. Um sicherzustellen, dass nur konstruktiv vorgegebene, zulässige Mengen an Injektionsflüssigkeit ausgepresst werden können, ist eine Sperrkontur vorgesehen, die mit dem Zustellteil zusammenwirkt. Die Sperrkontur stellt sicher, dass das Zustellteil nur in konstruktiv vorgegebenen Stellungen in Richtung der Längsmittelachse bewegt werden kann, ohne sich dabei gegenüber dem Gehäuse zu drehen. Die Sperrkontur verläuft dabei spiralförmig um die Längsmittelachse des Injektionsgeräts. Der Verlauf der Sperrkontur entspricht dabei der Bewegung des Zustellteils, so dass das Zustellteil bei seiner schraubenlinienförmigen Bewegung in distaler Richtung, die das Zustellteil beim Einstellen der auszupressenden Menge an Injektionsflüssigkeit ausführt, an der Sperrkontur anliegt. Durch die Unterbrechung der Sperrkontur wird ein Auspressen von Injektionsflüssigkeit ermöglicht. Die Injektionsflüssigkeit kann dabei nur in Mengeneinheiten abgegeben werden, die der Lage der Unterbrechung der Sperrkontur zugeordnet sind. Dabei kann nur eine einzige Unterbrechung der Sperrkontur vorgesehen sein. Es können jedoch auch mehrere Unterbrechungen der Sperrkontur vorgesehen sein, die vorteilhaft einen konstanten Winkelabstand zueinander um die Längsmittelachse besitzen. So können beispielsweise zwei Unterbrechungen an der Sperrkontur vorgesehen sein, die um 180° um die Längsmittelachse zueinander versetzt sind. Auch vier jeweils um 90° zueinander beabstandete Unterbrechungen können vorteilhaft sein. Die Anzahl der Unterbrechungen ist abhängig vom Anwendungsfall. Die Sperrkontur erstreckt sich vorteilhaft von der Position, in der das Zustellteil vor dem Einstellen der Dosis steht, bis zu der Position, in der sich das Zustellteil befindet, wenn die maximal mögliche Dosis eingestellt ist. Dadurch, dass die Sperrkontur unabhängig von Raststellungen des Bedienelements ist, kann eine gewünschte Raststärke und ein gewünschter Rastabstand unabhängig von den Mengeneinheiten an Injektionsflüssigkeit, die ausgepresst werden können, vorgesehen werden. Die Bedieneigenschaften des Injektionsgeräts sind dadurch weitgehend unabhängig von den injizierbaren Flüssigkeitsmengen konstruktiv einstellbar.

Die "proximale Richtung" bezeichnet dabei die Injektionsrichtuhg, also in Richtung zu einer Aufnahme für die Injektionsnadel hin bzw. die Richtung, in der die Injektionsflüssigkeit aus dem Behälter ausgepresst wird. Die "distale Richtung" bezeichnet die entgegengesetzte Richtung, also von der Injektionsnadel weg. Das distale Ende des Injektionsgeräts ist das der Injektionsnadel abgewandt liegende Ende. Mit "proximal" wird die Seite des Injektionsgeräts bezeichnet, die bei einer Injektion der Einstichstelle zugewandt liegt und mit "distal" die Seite, die der Einstichstelle abgewandt liegt.

Das Bedienelement ist beim Einstellen der auszupressenden Menge an Injektionsflüssigkeit vorteilhaft in mindestens eine Injektionsstellung, in der eine zulässige Menge an Injektionsflüssigkeit eingestellt ist, und in mindestens eine Sperrstellung, in der eine unzulässige Menge an Injektionsflüssigkeit eingestellt ist, stellbar. In Sperrstellung liegt das Zustellteil vorteilhaft mit einem Sperrelement an der Sperrkontur an. Das Sperrelement und die Sperrkontur überlappen sich in axialer Richtung und verhindern so eine Bewegung des Zustellteils in Richtung der Längsmittelachse in proximaler Richtung. In Injektionsstellung ist das Sperrelement vorteilhaft an der distalen Seite der Unterbrechung der Sperrkontur angeordnet. Die Unterbrechung ist so ausgebildet, dass sie sich in axialer Richtung des Injektionsgeräts nicht mit dem Sperrelement überlappt, so dass die Bewegung des Zustellteils mit dem Sperrelement in proximaler Richtung im Bereich der Unterbrechung nicht behindert wird.

Vorteilhaft besitzt das Injektionsgerät eine Rasteinrichtung, die in Injektionsstellung einrastet und die zwischen Zustellteil und Gehäuse wirkt. Dadurch wird dem Bediener angezeigt, wann die zulässige Injektionsstellung erreicht ist. Durch die Ausbildung der Rasteinrichtung zwischen Zustellteil und Gehäuse wird ein einfacher Aufbau erreicht. Die Rasteinrichtung ist dadurch nur beim Einstellen der auszupressenden Menge an Injektionsflüssigkeit, nicht jedoch beim Injektionsvorgang aktiv. Dadurch kann die für den Injektionsvorgang benötigte Kraft gering gehalten werden. Es kann jedoch vorteilhaft sein, eine beim Injektionsvorgang wirkende Rasteinrichtung vorzusehen. Vorteilhaft besitzt das Zustellteil mindestens einen Rastarm, der an der Sperrkontur anliegt und das Sperrelement bildet. Das Injektionsgerät besitzt vorteilhaft mindestens eine Rasterhöhung, die in Umfangsrichtung benachbart zu der Unterbrechung der Sperrkontur angeordnet ist und die mit dem Rastarm die Rasteinrichtung bildet. Um sicherzustellen, dass sich das Zustellteil beim Injektionsvorgang nicht gegenüber dem Gehäuse drehen kann, ist vorgesehen, dass das Injektionsgerät eine Längsführung besitzt, die das Zustellteil bei seiner Bewegung in proximaler Richtung drehfest mit dem Gehäuse verbindet. Eine einfache Gestaltung ergibt sich, wenn das Injektionsgerät mindestens einen Längssteg besitzt, der parallel zur Längsmittelachse des Injektionsgeräts verläuft und der die Rasterhöhung und die Längsführung für den Rastarm bildet. Vorteilhaft sind in Umfangsrichtung beidseitig zu der Unterbrechung Längsführungen angeordnet, so dass die Injektionsstellung in beiden Drehrichtungen als Rastposition definiert ist.

Die Drehbewegung des Zustellteils beim Einstellen der auszupressenden Menge an Injektionsflüssigkeit, die sich aufgrund der drehfesten Verbindung mit dem vom Bediener zu drehenden Bedienelement ergibt, bewirkt vorteilhaft über eine erste Gewindeverbindung die axiale Bewegung des Zustellteils. Das Zustellteil wird dabei um einen ersten Stellweg in distaler Richtung verschoben. Die erste Gewindeverbindung umfasst vorteilhaft ein Innengewinde des Zustellteils, das mit einem Außengewinde eines drehfest mit dem Gehäuse verbundenen Dosierkolbens zusammenwirkt. Da sowohl das Zustellteil als auch der Dosierkolben beim Auspressen der Injektionsflüssigkeit aus dem Behälter drehfest im Gehäuse gehalten sind, stellt die erste Gewindeverbindung beim Auspressen der Injektionsflüssigkeit eine feste Kopplung zwischen Zustellteil und Dosierkolben dar, über die die axiale Bewegung des Zustellteils in proximaler Richtung auf den Dosierkolben übertragen wird.

Die distale Bewegung des Bedienelements beim Einstellen der auszupressenden Menge an Injektionsflüssigkeit wird vorteilhaft über eine zweite Gewindeverbindung bewirkt.

Das Bedienelement ist vorteilhaft mehrteilig ausgebildet und besitzt einen Betätigungsknopf und eine Stellhülse. Der Betätigungsknopf ist vorteilhaft über einen Mitnehmer mit dem Zustellteil verbunden. Die Stellhülse ist insbesondere fest mit dem Dosierorgan verbunden. Der Betätigungsknopf kann vorteilhaft gegenüber der Stellhülse zwischen einer ersten, distalen Position und einer zweiten, proximalen Position verschoben werden. Der Betätigungsknopf ist insbesondere über eine Kupplung mit der Stellhülse verbunden, wobei die Kupplung in der ersten, distalen Position des Betätigungsknopfs eine drehfeste Verbindung zwischen Mitnehmer und Stellhülse herstellt und in der zweiten, proximalen Position des Betätigungsknopfs eine Drehung der Stellhülse gegenüber dem Mitnehmer zulässt Der Betätigungsknopf ist insbesondere in Richtung auf seine distale Position gefedert, so dass der Bedienknopf in seiner distalen Position steht, wenn der Bediener den Bedienknopf nicht in proximale Richtung drückt. In der ersten, distalen Position befindet sich der Bedienknopf beim Einstellen der auszupressenden Menge an Injektionsflüssigkeit. In dieser Position des Betätigungsknopfs werden Stellhülse und Mitnehmer vom Bediener gemeinsam gedreht. Dabei drehen sich auch das Dosierorgan und das Zustellteil. In der zweiten, proximalen Position des Betätigungsknopfs kann sich die Stellhülse gegenüber dem Mitnehmer drehen. Diese Position des Betätigungsknopfs wird erreicht, wenn der Bediener den Betätigungsknopf in proximale Richtung drückt, um eine Injektion auszuführen.

Das Dosierorgan ist vorteilhaft über die zweite Gewindeverbindung mit dem Gehäuse verbunden. Die Drehbewegung des Dosierorgans bewirkt insbesondere eine Bewegung des Dosierorgans und des Bedienelements in distaler Richtung um einen zweiten Stellweg. Beim Einstellen der auszupressenden Menge an Injektionsflüssigkeit bewegt sich das Bedienelement demnach in distaler Richtung. Das Dosierorgan bewegt sich entlang einer Schraubenlinie in distaler Richtung aus dem Gehäuse. Der zweite Stellweg ist dabei vorteilhaft größer als der erste Stellweg. Dadurch, dass der Stellweg für das Bedienelement deutlich größer als der Stellweg des Zustellteils ist, wird zum Auspressen der Injektionsflüssigkeit nur eine verringerte Kraft benötigt. Gleichzeitig ist für den Bediener aufgrund des großen Wegs des Bedienelements einfach erkennbar, ob die Injektionsflüssigkeit bereits ausgepresst wurde oder nicht.

Vorteilhaft besitzt das Injektionsgerät einen Schieber, der ein Gewinde einer dritten Gewindeverbindung trägt. Vorteilhaft ist der Schieber drehfest mit dem Dosierorgan verbunden und über die dritte Gewindeverbindung mit dem Gehäuse. Die dritte Gewindeverbindung bewirkt bei einer Drehbewegung des Dosierorgans und damit des Schiebers relativ zum Gehäuse eine Bewegung des Schiebers in distaler Richtung der Längsmittelachse um einen dritten Stellweg. Der dritte Stellweg ist dabei vorteilhaft genau so groß wie der erste Stellweg. Alternativ kann jedoch auch vorgesehen sein, dass der Schieber drehfest mit dem Gehäuse und über die dritte Gewindeverbindung mit dem Dosierorgan verbunden ist. Auch in diesem Fall bewirkt die dritte Gewindeverbindung bei einer Drehbewegung des Dosierorgans relativ zum Gehäuse eine dann allerdings rein axiale Bewegung des Schiebers in distaler Richtung der Längsmittelachse um einen dritten Stellweg. Auch hier ist der dritte Stellweg vorteilhaft genau so groß wie der erste Stellweg. Die dritte Gewindeverbindung besitzt eine Steigung, die bei Anordnung der dritten Gewindeverbindung zwischen Schieber und Gehäuse der Steigung der ersten Gewindeverbindung entspricht. Ist die dritte Gewindeverbindung zwischen dem Schieber und dem Mitriehmer ausgebildet, so entspricht die Steigung der dritten Gewindeverbindung der Differenz zwischen erster und zweiter Gewindeverbindung, so dass sich für den Schieber der gleiche Stellweg ergibt wie für das Zustellteil. Der Schieber besitzt vorteilhaft einen Mitnahmeabsatz, der mit einem Mitnahmeabsatz des Zustellteils zusammenwirkt und der eine axiale Bewegung des Schiebers in proximaler Richtung auf das Zustellteil überträgt. Über den Schieber und die dritte Gewindeverbindung bzw. die erste Gewindeverbindung und die dritte Gewindeverbindung erfolgt damit die Zustellbewegung zum Auspressen der auszupressenden Menge an Injektionsflüssigkeit.

Ein Ausführungsbeispiel der Erfindung wird im Folgenden anhand der Zeichnung erläutert. Es zeigen:
- Fig. 1: eine Seitenansicht eines Injektionsgeräts,
- Fig. 2: einen Schnitt entlang der Linie II-II in Fig. 1,
- Fig. 3: das Injektionsgerät aus Fig. 1 nach dem Einstellen der auszupressenden Menge an Injektionsflüssigkeit,
- Fig. 4: einen Schnitt entlang der Linie IV-IV in Fig. 3,
- Fig. 5: eine vergrößerte Darstellung des distalen Gehäuseteils des Injektionsgeräts aus Fig. 2,
- Fig. 6: eine ausschnittsweise Schnittdarstellung des Injektionsgeräts aus Fig. 4 im Bereich des Bedienelements nach dem Drücken des Betätigungsknopfs,
- Fig. 7: eine Seitenansicht des Mitnehmers des Injektionsgeräts,
- Fig. 8: einen Schnitt entlang der Linie VIII-VIII in Fig. 7,
- Fig. 9: einen Schnitt entlang der Linie IX-IX in Fig. 7,
- Fig. 10: eine Seitenansicht des Dosierorgans des Injektionsgeräts,
- Fig. 11: einen Schnitt entlang der Linie XI-XI in Fig. 10,
- Fig. 12: eine Seitenansicht des Dosierorgans in Richtung des Pfeils XII in Fig. 10,
- Fig. 13: eine Seitenansicht eines Innenrohrs des Injektionsgeräts,
- Fig. 14: einen Schnitt entlang der Linie XIV-XIV in Fig. 13,
- Fig. 15: einen Schnitt entlang der Linie XV-XV in Fig. 13,
- Fig. 16: eine vergrößerte Darstellung der Einzelheit XVI in Fig. 14,
- Fig. 17: eine Seitenansicht des Schiebers des Injektionsgeräts,
- Fig. 18: einen Schnitt entlang der Linie XVIII-XVIII in Fig. 17,
- Fig. 19: einen Schnitt entlang der Linie XIX-XIX in Fig. 17,
- Fig. 20: eine Seitenansicht des Zustellteils des Injektionsgeräts,
- Fig. 21: einen Schnitt entlang der Linie XXI-XXI in Fig. 20,
- Fig. 22: einen Schnitt entlang der Linie XXII-XXII in Fig. 20,
- Fig. 23: eine Seitenansicht des Zustellteils in Richtung des Pfeils XXIII in Fig. 20,
- Fig. 24: eine Seitenansicht der Kolbenstange des Dosierkolbens des Injektionsgeräts,
- Fig. 25: eine Seitenansicht in Richtung des Pfeils XXV in Fig. 24,
- Fig. 26: einen Schnitt durch das Innenrohr auf der Höhe der Linie XV-XV in Fig. 13 mit darin angeordnetem Zustellteil in Sperrstellung des Bedienelements,
- Fig. 27: eine Schnittdarstellung entsprechend Fig. 26 mit dem Zustellteil in Injektionsstellung des Bedienelements.

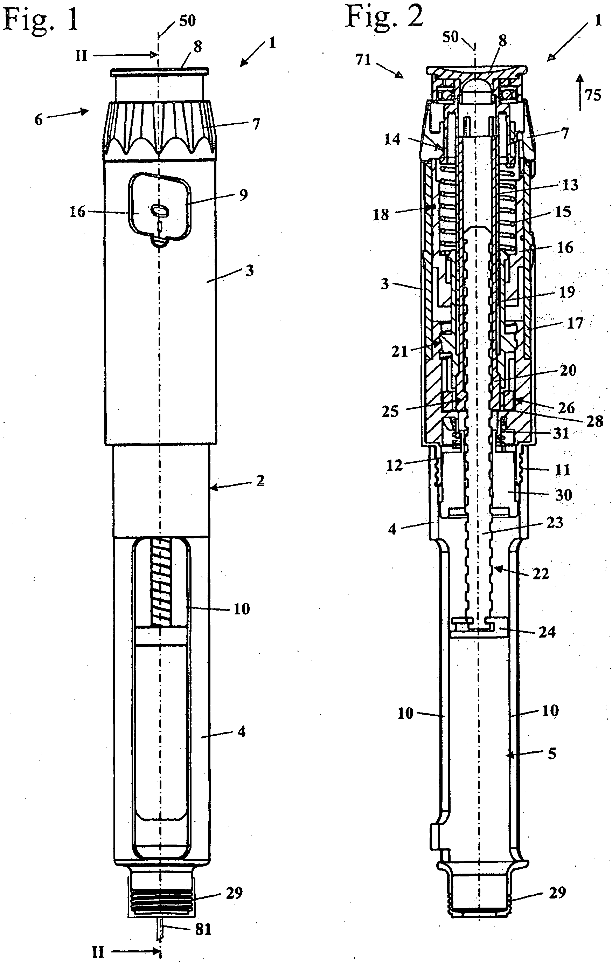

Das in Fig. 1 gezeigte Injektionsgerät 1 besitzt ein Gehäuse 2, das ein oberes, distales Gehäuseteil 3 und einen an der proximalen Seite des oberen Gehäuseteils 3 angeordneten,Halter 4 umfasst. An seinem proximalen Ende besitzt der Halter 4 ein Außengewinde 29, an dem eine in Fig. 1 schematisch gezeigte Injektionsnadel 81 aufgeschraubt werden kann. Im Halter 4 ist eine in Fig. 2 gezeigte Aufnahme 5 für einen Behälter mit Injektionsflüssigkeit ausgebildet. Der Behälter mit Injektionsflüssigkeit ist in den Figuren nicht gezeigt. Wie Fig. 1 zeigt, weist der Halter 4 mindestens eine Aussparung 10 auf, durch die der Behälter mit Injektionsflüssigkeit sichtbar ist. Dadurch kann der Bediener leicht erkennen, ob noch Injektionsflüssigkeit im Behälter vorhanden ist. Wie Fig. 2 zeigt, sind zwei einander gegenüberliegend angeordnete Aussparungen 10 am Halter 4 vorgesehen.

Wie Fig. 1 zeigt, ist am distalen Ende des Gehäuseteils 3 ein Bedienelement 6 angeordnet, das eine Stellhülse 7 und einen an der distalen Seite der Stellhülse 7 angeordneten Betätigungsknopf 8 besitzt. Benachbart zur Stellhülse 7 weist das Gehäuseteil 3 ein Sichtfenster 9 auf, durch das eine auf einem Dosierorgan 16 aufgebrachte Skala erkennbar ist. Das Dosierorgan 16 ist im Gehäuseteil 3 angeordnet. In Fig. 1 zeigt die Skala eine "0", die dem Bediener signalisiert, dass keine Mengeneinstellung vorgenommen wurde.

Fig. 2 zeigt den Aufbau des Injektionsgeräts 1 im Einzelnen. Das Injektionsgerät 1 besitzt einen Mitnehmer 13, der im Wesentlichen hülsenförmig ausgebildet ist und der axial fest mit dem Betätigungsknopf 8 des Bedienelements 6 verbunden ist. Der Begriff "axial" bezieht sich dabei jeweils auf die Richtung einer Längsmittelachse 50 des Injektionsgeräts 1. Der Betätigungsknopf 8 ist mit dem Mitnehmer 13 über eine Schnappverbindung verbunden, die eine Drehung des Betätigungsknopfs 8 gegenüber dem Mitnehmer 13 zulässt. Der Mitnehmer 13 ist über eine Kupplung 14 mit der Stellhülse 7 des Bedienelements 6 verbunden. Bei der in Fig. 2 gezeigten ersten, distalen Position 71 des Betätigungsknopfs 8 ist die Kupplung 14 geschlossen. Die Stellhülse 7 des Bedienelements 6 ist drehfest mit dem Mitnehmer 13 verbunden. Die Stellhülse 7 ist fest mit einem Dosierorgan 16 verbunden, das auch als Einstellglied oder Skalenrohr bezeichnet wird. Der Mitnehmer 13 ist drehfest mit einem Zustellteil 20 verbunden, das über eine erste Gewindeverbindung 25 mit einer Kolbenstange 23 eines Dosierkolbens 22 verbunden ist. Die Kolbenstange 23 trägt an ihrem proximalen Ende eine Kolbenscheibe 24, die zur Anlage an einem Stopfen des Behälters mit Injektionsflüssigkeit dient und über die die Injektionsflüssigkeit aus dem Behälter ausgepresst wird.

Die Kolbenstange 23 ist drehfest in einem Kolbenstangenring 30 gehalten. Der Kolbenstangenring 30 ist axial verschiebbar im Injektionsgerät 1 angeordnet. In der in Fig. 2 gezeigten Stellung, wenn kein Behälter in die Aufnahme 5 eingesetzt ist, wird der Kolbenstangenring 30 von einer Druckfeder 31 in seine proximale Position gedrückt. In dieser Position ist der Kolbenstangenring 30 gegenüber dem Gehäuseteil 3 drehbar. Wird ein Behälter in die Aufnahme 5 eingesetzt und der Halter 4 an einem Befestigungsgewinde 11 mit dem Gehäuseteil 3 verbunden, so drückt der Behälter den Kolbenstangenring 30 in distale Richtung. Das Injektionsgerät 1 besitzt ein Innenrohr 17, das drehfest mit dem Gehäuseteil 3 verbunden ist. An seinem distalen Ende weist der Kolbenstangenring 30 eine Kontur 12 auf, die auf eine Kontur des Innenrohrs 17 abgestimmt ist. In seiner distalen Stellung ist der Kolbenstangenring 30 über die genannten Konturen drehfest mit dem Innenrohr 17 und damit auch drehfest mit dem Gehäuseteil 3 verbunden. Bei in die Aufnahme 5 eingesetztem Behälter ist dadurch die Kolbenstange 23 drehfest im Gehäuseteil 3 gehalten. Durch die drehfeste Verbindung von Kolbenstange 23 und Gehäuseteil 3 bewirkt eine Drehung des Zustellteils 20 eine Bewegung des Zustellteils 20 in distaler Richtung, also in Richtung des Pfeils 75 in Fig. 2. Zwischen dem Zustellteil 20 und dem Innenrohr 17 ist eine Rasteinrichtung 26 gebildet, die Raststellungen des Zustellteils 20 definiert. Bei der in Fig. 2 gezeigten Stellung des Zustellteils 20 liegt das Zustellteil 20 an einem am Innenrohr 17 gebildeten Anschlag 28 an, der die Position des Zustellteils 20 in axialer Richtung definiert.

Das Dosierorgan 16 ist über eine zweite Gewindeverbindung 18 mit dem Innenrohr 17 verbunden. Das Innenrohr 17 ist fest mit dem Gehäuseteil 3 verbunden. Das Innenrohr könnte auch einteilig mit dem Gehäuseteil 3 ausgebildet sein, allerdings wird dadurch die Herstellung des Injektionsgeräts 1 sehr aufwendig. Das Dosierorgan 16 ist drehfest und axial verschiebbar mit einem Schieber 19 verbunden, der ins Innere des Dosierorgans 16 ragt. Der Schieber 19 ist über eine dritte Gewindeverbindung 21 mit dem Innenrohr 17 verbunden. Zwischen dem Mitnehmer 13 und dem Dosierorgan 16 wirkt eine Druckfeder 15, die den Betätigungsknopf 8 in seine erste Position 71 drückt.

Zum Einstellen der auszupressenden Menge an Injektionsflüssigkeit dreht der Bediener das Bedienelement 6, bis die gewünschte Dosis im Sichtfenster 9 erscheint. Dabei dreht sich die Stellhülse 7. Das drehfest mit der Stellhülse 7 verbundene Dosierorgan 16 dreht sich dadurch gegenüber dem oberen Gehäuseteil 3 und dem Innenrohr 17. Aufgrund seiner Drehbewegung wird das Dosierorgan 16 über die zweite Gewindeverbindung 18 in distaler Richtung, also in Richtung des Pfeils 75 verschoben. Das Bedienelement 6 und der axial fest mit dem Betätigungsknopf 8 des Bedienelements 6 verbundene Mitnehmer 13 bewegen sich mit dem Dosierorgan 16. Das Bedienelement 6, der Mitnehmer 13 und das Dosierorgan 16 bewegen sich gemeinsam in distaler Richtung und drehen sich dabei aufgrund der zweiten Gewindeverbindung 18 um die Längsmittelachse 50.

Über die drehfeste Verbindung zwischen Mitnehmer 13 und Zustellteil 20 wird auch das Zustellteil 20 gegenüber dem oberen Gehäuseteil 3 gedreht. Über die erste Gewindeverbindung 25 bewegt sich auch das Zustellteil 20 in distaler Richtung. Der Schieber 19 bewegt sich ebenfalls in distaler Richtung, da der Schieber 19 drehfest mit dem Dosierorgan 16 verbunden ist. Auch der Schieber 19 und das Zustellteil 20 bewegen sich mit einer kombinierten Dreh- und Längsbewegung, wobei der Weg, den Schieber 19 und Zustellteil 20 in Richtung der Längsmittelachse 50 zurücklegen, über die erste Gewindeverbindung 25 bzw. über die dritte Gewindeverbindung 21 festgelegt sind. Es kann auch vorgesehen sein, dass der Schieber 19 über eine dritte Gewindeverbindung mit dem Dosierorgan 16 verbunden ist und drehfest mit dem Zustellteil 20 verbunden ist.

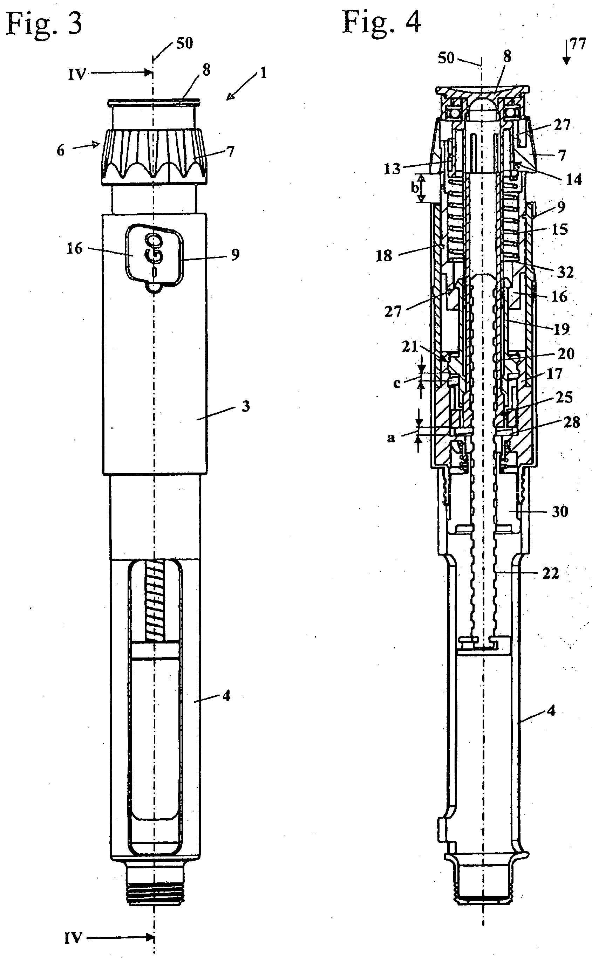

Die Figuren 3 und 4 zeigen das Injektionsgerät 1 nach dem Einstellen der auszupressenden Menge an Injektionsflüssigkeit. Das Zustellteil 20 hat sich um einen ersten Stellweg a in distaler Richtung bewegt. Nach dem Einstellen der auszupressenden Menge an Injektionsflüssigkeit hat sich die Stirnseite des Zustellteils 20 um den ersten Stellweg a vom Anschlag 28 entfernt. Das Bedienelement 6 mit der Stellhülse 7 und dem Betätigungsknopf 8 hat sich um einen zweiten Stellweg b in distaler Richtung bewegt. Der zweite Stellweg b ist im Ausführungsbeispiel zwischen der proximalen Stirnseite der Stellhülse 7 und der distalen Stirnseite des Gehäuseteils 3 gemessen. Der zweite Stellweg b ist deutlich größer als der erste Stellweg a. Im Ausführungsbeispiel beträgt der zweite Stellweg b ein Vielfaches, beispielsweise etwa das 3fache des Stellwegs a. Die unterschiedlichen Stellwege a und b ergeben sich durch unterschiedliche Steigungen der ersten Gewindeverbindung 25 und der zweiten Gewindeverbindung 18. Der Schieber 19 hat sich um einen dritten Stellweg c in distaler Richtung bewegt. Der dritte Stellweg c ist in Fig. 4 an der proximalen Stirnseite des Abschnitts des Schiebers 19, der das Gewinde trägt, eingezeichnet, und zwar gegenüber der Position dieser Stirnseite in Fig. 2.

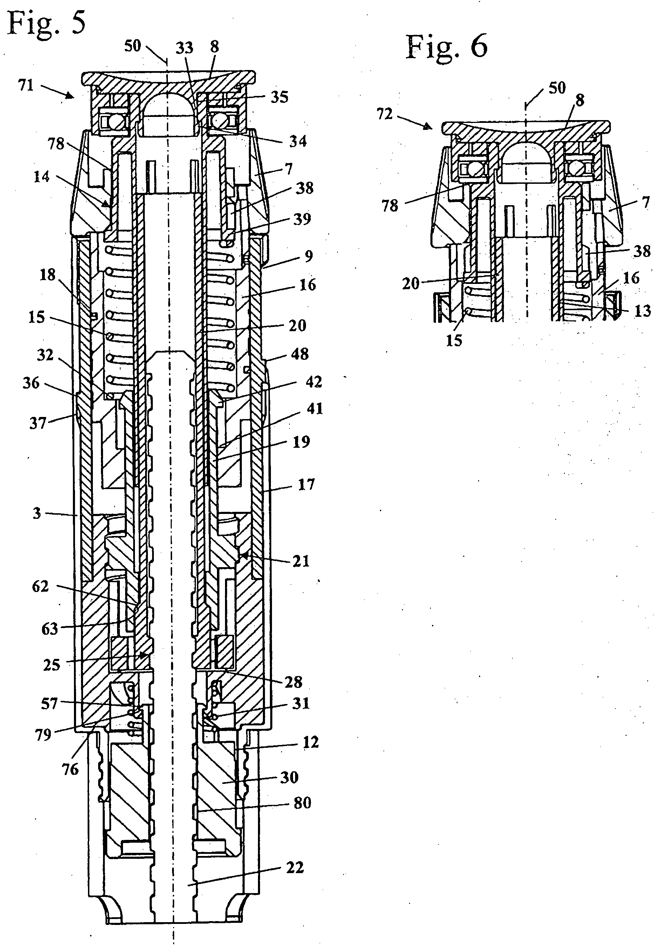

Die maximal einzustellende Menge an Injektionsflüssigkeit ist durch den Weg vorgegeben, den der Betätigungsknopf 6 und das Dosierorgan 16 sich in distaler Richtung bewegen können. Dieser Weg wird durch einen zwischen dem Dosierorgan 16 und dem Schieber 19 gebildeten Anschlag 27 (Fig. 4) begrenzt. Wie Fig. 5 zeigt, besitzt das Dosierorgan 16 an seinem proximalen Ende einen nach innen gerichteten Absatz 41. Dieser Absatz 41 wird von einem Rastrand 42 am Schieber 19 in axialer Richtung hintergriffen. Der Absatz 41 bildet mit dem Rastrand 42 den Anschlag 27. Sobald der Rastrand 42 am Absatz 41 anliegt, ist die maximal einstellbare Menge an Injektionsflüssigkeit erreicht. Der Abstand zwischen Absatz 41 und Rastrand 42 bei dem in den Figuren 1 und 2 gezeigten Zustand des Injektionsgeräts 1 entspricht dem zweiten Stellweg b abzüglich dem ersten Stellweg a.

Wie Fig. 5 zeigt, besitzt das erste Gehäuseteil 3 eine Rastvertiefung 37, die im Ausführungsbeispiel umlaufend ausgebildet ist. In die Rastvertiefung 37 ragt eine am Innenrohr 17 ausgebildete, radial nach außen ragende Raste 36, die das Innenrohr 17 in Richtung der Längsmittelachse 50 des Injektionsgeräts 1 im Gehäuseteil 3 sichert. An seinem proximalen Ende liegt das Innenrohr 17 an einer Schulter 76 des Gehäuseteils 3 an. Zur Drehlagensicherung besitzt das Innenrohr 17 einen nach außen ragenden Zapfen 48, der benachbart zum Sichtfenster 9 am Gehäuseteil 3 einrastet.

Fig. 5 zeigt auch die Abstützung der Druckfeder 15. Die Druckfeder 15 stützt sich mit ihrem proximalen Ende an einer Schulter 32 des Dosierorgans 16 und mit ihrem distalen Ende an einem am Mitnehmer 13 ausgebildeten Rand 39 ab. Der Rand 39 ragt von einem hülsenförmigen Abschnitt des Mitnehmers 13 nach außen. Benachbart zum Rand 39 ist an der distalen Seite des Rands 39 eine Außenverzahnung 38 am Mitnehmer 13 angeordnet. Die Außenverzahnung 38 wirkt mit einer nicht gezeigten Innenverzahnung an der Stellhülse 7 zusammen und bildet mit dieser die Kupplung 14. Bei der in Fig. 5 gezeigten, nicht betätigten Stellung des Betätigungsknopfs 8 ist die Kupplung 14 geschlossen und stellt eine drehfeste Verbindung zwischen Mitnehmer 13 und Stellhülse 7 her. Die Druckfeder 15 drückt den Mitnehmer 13 in Richtung auf die geschlossene Stellung der Kupplung 14. Dadurch wird der Betätigungsknopf 8 in Richtung auf seine distale Position 71 gedrückt.

Nach dem Einstellen der zu injizierenden Dosis kann eine Injektion ausgelöst werden. Hierzu wird der Betätigungsknopf 8 in Richtung des Pfeils 77 in Fig. 4, also in proximaler Richtung, gedrückt. Dadurch bewegt sich der Betätigungsknopf 8 entgegen der Kraft der Druckfeder 15 in Richtung der Längsmittelachse 50 in die Stellhülse 7 hinein, bis der Betätigungsknopf 8 an einem Anschlag 78 der Stellhülse 7 anliegt. Fig. 6 zeigt den Betätigungsknopf 8 in seiner zweiten, proximalen Position 72. In dieser Position hat sich die Außenverzahnung 38 des Mitnehmers 13 aus dem Bereich der Stellhülse 7 bewegt. Dadurch ist die Stellhülse 7 gegenüber Mitnehmer 13 und dem Betätigungsknopf 8 drehbar. Die Kupplung 14 ist offen. Bei weiterem Drücken des Betätigungsknopfs 8 in Richtung des Pfeils 77 in Fig. 4 wird das Dosierorgan 16 in das Innenrohr 17 gedrückt und verschiebt sich dabei in proximaler Richtung. Dabei dreht sich das Dosierorgan 16 aufgrund der zweiten Gewindeverbindung 18. Aufgrund der Drehung des Dosierorgans 16 wird auch der Schieber 19 gedreht und bewegt sich dadurch in proximaler Richtung. Der Schieber 19 besitzt einen Mitnahmeabsatz 62, der an einem Mitnahmeabsatz 63 des Zustellteils 20 anliegt. Über die Mitnahmeabsätze 62 und 63 drückt der Schieber 19 bei seiner Bewegung in proximaler Richtung auf das Zustellteil 20 und bewegt dieses ebenfalls in proximaler Richtung. Das Zustellteil 20 ist drehfest mit dem Mitnehmer 13 verbunden, der mit dem nicht rotierenden Betätigungsknopf 8 axial fest verbunden ist. Da das Zustellteil 20 nicht rotiert und auch der Dosierkolben 22 über den Kolbenstangenring 30 drehfest mit dem Gehäuseteil 3 verbunden ist, sind das Zustellteil 20 und der Dosierkolben 22 fest miteinander verbunden und bewegen sich gemeinsam in proximaler Richtung, bis das Zustellteil 20 am Anschlag 28 anliegt und die eingestellte Menge an Injektionsflüssigkeit vollständig aus dem Behälter ausgepresst wurde.

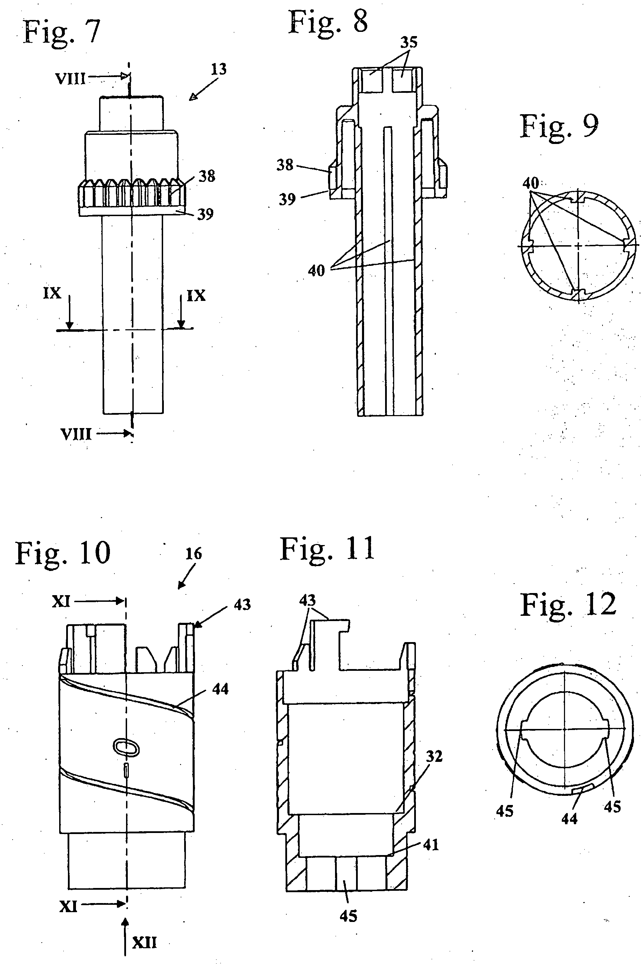

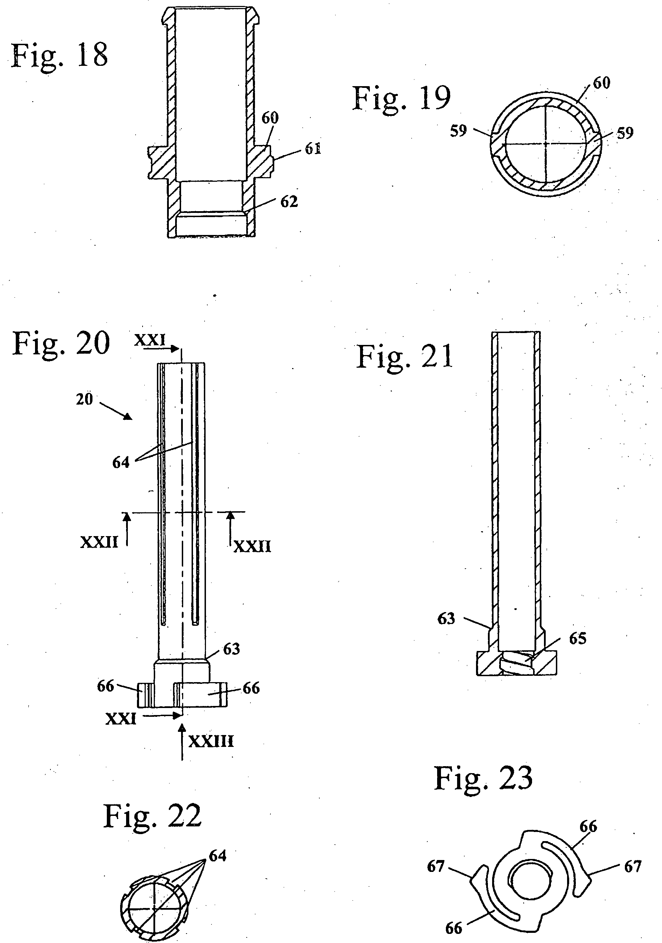

Die Figuren 7 bis 25 zeigen die Bauteile des Injektionsgeräts 1 im Einzelnen. In den Figuren 7 bis 9 ist der Mitnehmer 13 gezeigt. Zur Verbindung mit dem Betätigungsknopf 8 besitzt der Mitnehmer 13 an seinem distalen Ende innenliegende Rasterhöhungen 35, die einen an einem Stutzen 33 des Betätigungsknopfs 8 gebildeten, in Fig. 5 gezeigten Rastrand 34 hintergreifen und dadurch den Betätigungsknopf 8 in axialer Richtung mit dem Mitnehmer 13 verbinden. Der hülsenförmige Mitnehmer 13 besitzt an seinem Innenumfang im Ausführungsbeispiel vier in axialer Richtung verlaufende Führungsstege 40. Die Führungsstege 40 sind auf in Fig. 20 gezeigte Längsnuten 64 des Zustellteils 20 angepasst und greifen in diese ein. Die Führungsstege 40 stellen mit den Längsnuten 64 die drehfeste Verbindung zwischen Mitnehmer 13 und Zustellteil 20 her. Die Führungsstege 40 sind in Richtung der Längsmittelachse 50 des Injektionsgeräts 1 in den Längsnuten 64 frei verschiebbar.

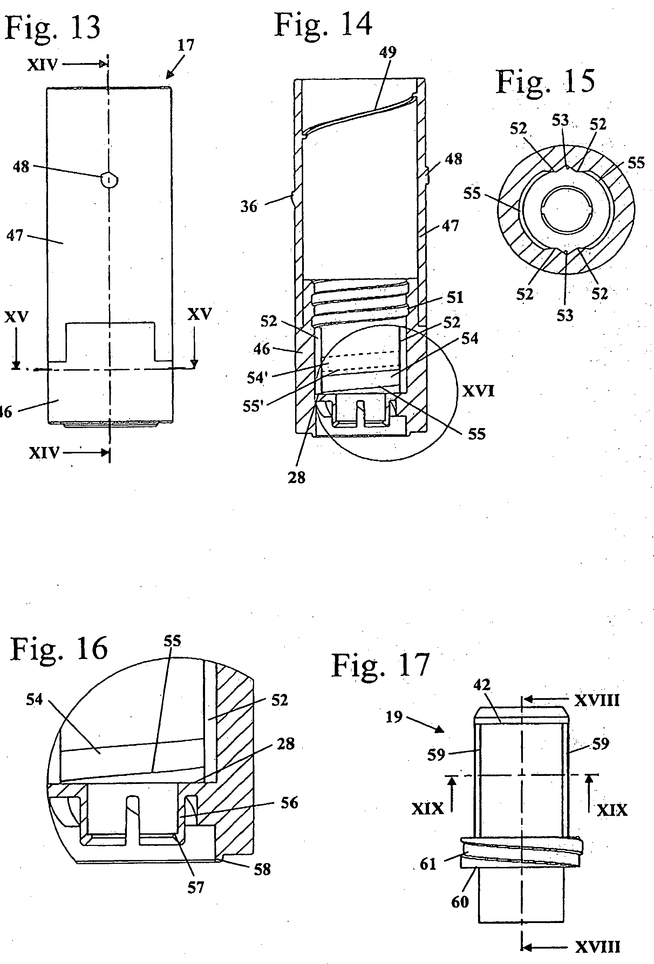

Die Figuren 10 bis 12 zeigen das Dosierorgan 16, das auch als Skalenrohr oder Einstellglied bezeichnet wird. Das Dosierorgan 16 ist hülsenförmig ausgebildet und besitzt an seinem Außenumfang ein Außengewinde 44. Das Außengewinde 44 ist als wendelförmig am Außenumfang des Dosierorgans 16 verlaufende Nut ausgebildet. An seinem distalen Ende trägt das Dosierorgan 16 eine Verbindungskontur 43, die aus haken- und rampenförmigen Elementen gebildet ist, die eine drehfeste Verbindung mit der Stellhülse 7 herstellen. Wie die Figuren 11 und 12 zeigen, besitzt das Dosierorgan 16 an seinem proximalen Ende zwei Führungsnuten 45, die parallel zur Längsmittelachse 50 verlaufen. Die Führungsnuten 45 sind einander gegenüberliegend angeordnet und wirken mit Längsstegen 59 des Schiebers 19 zusammen, die in den Figuren 17 und 19 gezeigt sind. Über die Längsstege 59, die in den Führungsnuten 45 geführt sind, ergibt sich eine drehfeste Verbindung zwischen Dosierorgan 16 und Schieber 19. Die Längsstege 59 sind in Richtung der Längsmittelachse 50 frei in den Führungsnuten 45 beweglich, so dass der Schieber 19 gegenüber dem Dosierorgan 16 in Richtung der Längsmittelachse 50 verschiebbar ist.

In den Figuren 13 bis 16 ist das Innenrohr 17 gezeigt. Das Innenrohr 17 ist zweiteilig ausgeführt und besteht aus einem proximalen Teil 46 und einem distalen Teil 47, die fest miteinander verbunden sind. Das Innenrohr 17 kann auch einteilig hergestellt werden. Allerdings ergibt sich dadurch eine deutlich aufwendigere Herstellung des Innenrohrs 17. Um die Herstellung weiter zu vereinfachen, kann es vorteilhaft sein, das Innenrohr 17 aus mehr als zwei Einzelteilen auszubilden.

Im distalen Teil 47 des Innenrohrs 17 ist ein Innengewinde 49 angeordnet, das aus einem spiralförmig am Innenumfang verlaufenden Steg gebildet ist. Das Innengewinde 49 ist durch einen einzigen Gewindegang gebildet. Es kann vorgesehen sein, das Innengewinde 49 nur durch einen oder mehrere Teilabschnitte eines Gewindegangs auszubilden. Das Innengewinde 49 wirkt mit dem Außengewinde 44 des Dosierorgans 16 zusammen und bewirkt eine axiale Verschiebung des Dosierorgans 16 bei einer Drehung des Dosierorgans 16. Im proximalen Teil 46 des Innenrohrs 17 ist ein Innengewinde 51 ausgebildet, das mit einem in Fig. 17 gezeigten Außengewinde 61 des Schiebers 19 zusammenwirkt. Das Innengewinde 51 bildet mit dem Außengewinde 61 die dritte Gewindeverbindung 21. An der proximalen Seite des Innengewindes 51 schließen sich Längsstege 52 an. Wie Fig. 15 zeigt, sind insgesamt vier Längsstege 52 vorgesehen, die jeweils beidseitig einer Unterbrechung 53 angeordnet sind. Im Ausführungsbeispiel sind zwei Unterbrechungen 53 vorgesehen, die einander gegenüberliegend, also in einem Abstand von einem Umfangswinkel von 180° zueinander, angeordnet sind.

Am proximalen Ende des Innenraums des Innenrohrs 17, in distaler Richtung an den Anschlag 28 anschließend, ist eine spiralförmige Nut 54 am Innenrohr 17 ausgebildet, die als Vertiefung in der Wand des Innenrohrs 17 ausgebildet ist. Eine weitere, in der Darstellung nicht sichtbare Nut 54 ist in der Halbschale des Innenrohrs vorgesehen, die in Fig. 14 vor der Schnittebene liegt. Die spiralförmige Nut 54 erstreckt sich im Ausführungsbeispiel über einen halben Gewindegang. Die Nut 54 kann sich jedoch auch über einen oder mehrere Gewindegänge erstrecken, wie in Fig. 14 schematisch durch die Nut 54' angedeutet ist, die gestrichelt eingezeichnet ist. Die proximale Wand der Nut 54 bildet eine Sperrkontur 55. Entsprechend bildet eine Wand der Nut 54' eine Sperrkontur 55'. Die Sperrkontur 55 wird im Folgenden noch näher beschrieben.

Wie Fig. 16 zeigt, ragt am proximalen Ende des Innenrohrs 17 ein Zentrierrand 58 in proximale Richtung. Der Zentrierrand 58 ragt in eine proximale Öffnung des Gehäuseteils 3 und stellt einen festen Sitz des Innenrohrs 17 im Gehäuseteil 3 sicher. An der proximalen Seite des Innenrohrs 17 ragen außerdem Haltestutzen 56 in proximale Richtung, an deren proximalen Ende radial nach innen ragende Rastränder 57 angeformt sind. Die Rastränder 57 wirken mit einem Rastrand 79 des Kolbenstangenrings 30 zusammen, der in Fig. 5 gezeigt ist. Der Rastrand 79 stellt mit dem Rastrand 57 eine axiale Sicherung für den Kolbenstangenring 30 dar. Wie Fig. 5 zeigt, drückt die zweite Druckfeder 31 den Kolbenstangenring 30 in seine proximale Position, bis der Rastrand 79 am Rastrand 57 anliegt. In dieser Position kann der Bediener das Gehäuseteil 3 gegenüber dem Kolbenstangenring 30 drehen, um den Dosierkolben 22 in distale Richtung zu bewegen. Dies ist beim Auswechseln eines Behälters für Injektionsflüssigkeit vorgesehen.

In den Figuren 17 bis 19 ist der Schieber 19 im Einzelnen gezeigt. An seinem distalen Ende besitzt der Schieber 19 den Rastrand 42. Wie die Figuren 17 und 18 zeigen, ist das Außengewinde 61 in einem Ringsteg 60 ausgebildet, der radial nach außen ragt. Auch der Schieber 19 ist im Wesentlichen hülsenförmig ausgebildet.

Die Figuren 20 bis 23 zeigen das Zustellteil 20. An seinem proximalen Ende besitzt das Zustellteil 20 zwei Rastarme 66, die in Fig. 23 gezeigt sind. An ihrem freien Ende besitzen die Rastarme 66 jeweils eine Raste 67, die radial nach außen weist. Die Rastarme 66 verlaufen etwa in Umfangsrichtung und sind radial nach außen federnd ausgebildet. Fig. 21 zeigt ein am proximalen Ende des Zustellteils 20 ausgebildetes Innengewinde 65, das mit dem Dosierkolben 22 zusammenwirkt. Das Innengewinde 65 und die Rastarme 65 sind im gleichen Längsabschnitt des Zustellteils 20 angeordnet.

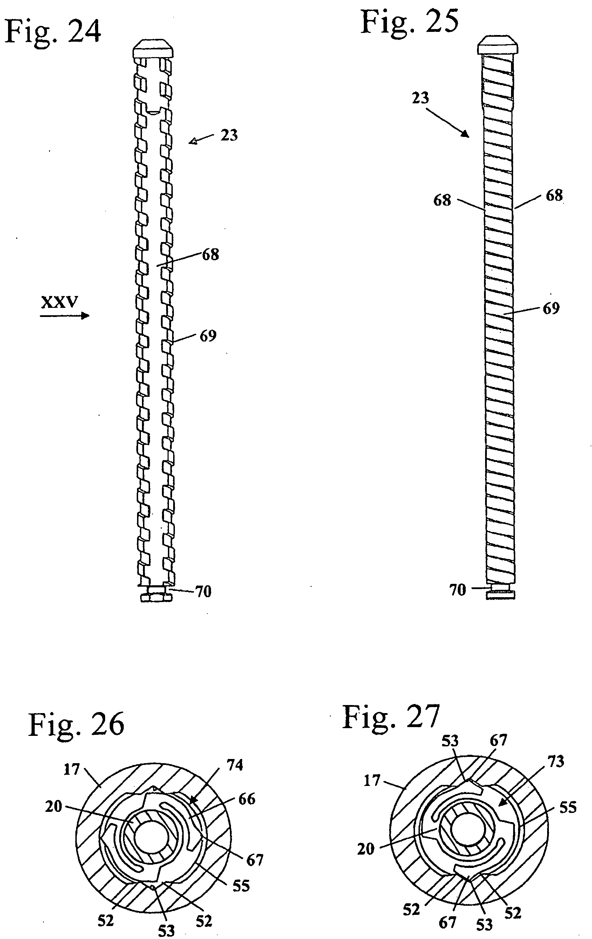

Wie die Figuren 24 und 25 zeigen, besitzt die Kolbenstange 23 ein Außengewinde 69, das mit dem Innengewinde 65 des Zustellteils 20 zusammenwirkt und mit diesem die erste Gewindeverbindung 25 bildet. An ihren gegenüberliegenden Längsseiten besitzt die Kolbenstange 23 Abflachungen 68, die zur Sicherung der Drehlage der Kolbenstange 23 mit entsprechenden Abflachungen einer in Fig. 5 gezeigten Öffnung 80 im Kolbenstangenring 30 zusammenwirkend An seinem proximalen Ende besitzt die Kolbenstange 23 eine Befestigungsnut 70, an der die Kolbenscheibe 24 gehalten ist.

Fig. 26 zeigt die Anordnung des Zustellteils 20 an der Sperrkontur 55 in einer Sperrstellung 74 von Bedienelement 6 und Zustellteil 20. In dieser Stellung liegen die Rasten 67 der Rastarme 66 an der Sperrkontur 55 an. Da sich die Rasten 67 und die Sperrkontur 55 in Richtung der Längsmittelachse 50 gesehen überlappen, kann das Zustellteil 20 nicht in proximaler Richtung verschoben werden. Die Sperrstellung 74 des Bedienelements 6 ist gegeben, wenn eine unzulässige Menge an Injektionsflüssigkeit eingestellt ist. Das Auspressen einer unzulässigen Menge an Injektionsflüssigkeit ist durch die Sperrkontur 55 konstruktiv verhindert.

Wird das Bedienelement 6 und damit auch das Zustellteil 20 weiter gedreht, so gelangen die Rasten 67 nach Überwinden der Längsstege 52, die die mit den Rasten 67 die Rasteinrichtung 26 bilden, in den Bereich von Unterbrechungen 53. An den Unterbrechungen 53 ist die Sperrkontur 55 unterbrochen. Die Unterbrechungen 53 sind als parallel zur Längsmittelachse 50 verlaufende Längsnuten ausgebildet. In Umfangsrichtung sind beidseitig der Unterbrechungen 53 Längsstege 52 angeordnet, so dass sich eine definierte Rastposition für das Zustellteil 20 in der in Fig. 27 gezeigten Injektionsstellung 73 ergibt. In Injektionsstellung 73 kann das Zustellteil 20 in proximale Richtung gedrückt werden. Dabei gleiten die Rasten 67 zwischen den Längsstegen 52, die parallel zur Längsmittelachse 50 verlaufen, in proximale Richtung. Beim Auslösen einer Injektion durch Drücken des Bedienknopfs 8 verhindern die Längsstege 52, dass sich das Zustellteil 20 während der Injektion dreht. Dadurch ist während der Injektion auch der Mitnehmer 13 drehfest im Gehäuse 2 gehalten und kann sich bei den während der Injektion herrschenden Kräften nicht mitdrehen.

Nur dann, wenn sich das Bedienelement 6 und damit auch das Zustellteil 20 in Injektionsstellung 73 befindet, kann der Bedienknopf 8 aus seiner proximalen Position 72 zusammen mit der Stellhülse 7, dem Dosierkolben 16, dem Schieber 19 und dem Zustellteil 20 in proximale Richtung verschoben werden. Dadurch wird die Kolbenstange 23 in proximale Richtung bewegt und Injektionsflüssigkeit aus dem Behälter ausgepresst. Befindet sich das Bedienelement 6 und damit das Zustellteil 20 in Sperrstellung 74, so kann der Betätigungsknopf 8 zwar in die Stellhülse 7 gedrückt werden, ein weiteres Verschieben des Betätigungsknopfs 8 gegenüber dem Gehäuseteil 3 in proximale Richtung ist jedoch nicht möglich, sondern wird aufgrund der Sperrkontur 55 gesperrt, die eine axiale Verschiebung des Dosierorgans 16, des Schiebers 19 und des Zustellteils 20 über die Rasten 67 der Rastarme 66 blockiert. Dadurch kann sichergestellt werden, dass nur eine konstruktiv vorgegebene, zulässige Menge an Injektionsflüssigkeit aus dem Behälter ausgepresst werden kann.

Im Ausführungsbeispiel ist ein Injektionsgerät 1 gezeigt, bei dem nur eine einzige vorgegebene Menge an Injektionsflüssigkeit aus dem Behälter ausgepresst werden kann. Diese Menge ist erreicht, wenn das Bedienelement um 180° gedreht wurde. Es kann jedoch auch vorgesehen sein, dass mehrere Injektionsstellungen 73 möglich sind, die unterschiedlichen Mengen an Injektionsflüssigkeit zugeordnet sind. Hierbei sind die Unterbrechungen 53 an den Stellen der Sperrkontur 55, die den gewünschten, zulässigen Mengen an Injektionsflüssigkeit zugeordnet sind, vorzusehen.

## Patentansprüche

1. Injektionsgerät mit einem Gehäuse (2), in dem eine Aufnahme (5) für einen Behälter mit Injektionsflüssigkeit ausgebildet ist, mit einem Bedienelement (6), das zur Einstellung einer aus dem Behälter auszupressenden Menge an Injektionsflüssigkeit drehbar ist und das sich beim Drehen in Richtung einer Längsmittelachse (50) des Injektionsgeräts (1) in distaler Richtung des Injektionsgeräts (1) gegenüber dem Gehäuse (2) bewegt, wobei das Bedienelement (6) zum Auspressen von Injektionsflüssigkeit aus dem Behälter in Richtung der Längsmittelachse (50) in proximaler Richtung des Injektionsgeräts (1) verschoben wird, wobei das Injektionsgerät (1) ein Zustellteil (20) besitzt, das beim Einstellen der auszupressenden Menge an Injektionsflüssigkeit drehfest mit dem Bedienelement (6) verbunden ist und in Richtung der Längsmittelachse (50) in distaler Richtung verschoben wird und das beim Auspressen von Injektionsflüssigkeit aus dem Behälter drehfest mit dem Gehäuse (2) verbunden ist und in Richtung der Längsmittelachse (50) in proximaler Richtung verschoben wird,
**dadurch gekennzeichnet, dass** das Injektionsgerät (1) eine spiralförmig um die Längsmittelachse (50) verlaufende Sperrkontur (55) besitzt, die mit dem Zustellteil (20) zusammenwirkt und die eine Bewegung des Zustellteils (20) in proximaler Richtung sperrt, wobei die Sperrkontur (55) an mindestens einer Stelle eine Unterbrechung (53) besitzt, die eine Bewegung des Zustellteils (20) in Richtung der Längsmittelachse (50) in proximaler Richtung zulässt.

2. Injektionsgerät nach Anspruch 1,
**dadurch gekennzeichnet, dass** das Bedienelement (6) beim Einstellen der auszupressenden Menge an Injektionsflüssigkeit in mindestens eine Injektionsstellung (73), in der eine zulässige Menge an Injektionsflüssigkeit eingestellt ist, und in mindestens eine Sperrstellung (74), in der eine unzulässige Menge an Injektionsflüssigkeit eingestellt ist, stellbar ist, wobei das Zustellteil (20) in Sperrstellung (74) an der Sperrkontur (55) anliegt und in Injektionsstellung (73) an der distalen Seite der Unterbrechung (53) der Sperrkontur (55) angeordnet ist.

3. Injektionsgerät nach Anspruch 2,
**dadurch gekennzeichnet, dass** das Injektionsgerät (1) eine Rasteinrichtung (26) besitzt, die in Injektionsstellung (73) einrastet und die zwischen Zustellteil (20) und Gehäuse (2) wirkt.

4. Injektionsgerät nach Anspruch 3,
**dadurch gekennzeichnet, dass** das Zustellteil (20) mindestens einen Rastarm (66) besitzt, der an der Sperrkontur (55) anliegt, und dass das Injektionsgerät (1) in Umfangsrichtung benachbart zu der Unterbrechung (53) der Sperrkontur (55) mindestens eine Rasterhöhung besitzt, die mit dem Rastarm (66) die Rasteinrichtung (26) bildet.

5. Injektionsgerät nach Anspruch 4,
**dadurch gekennzeichnet, dass** das Injektionsgerät eine Längsführung besitzt, die das Zustellteil (20) bei seiner Bewegung in proximaler Richtung drehfest mit dem Gehäuse (2) verbindet.

6. Injektionsgerät nach Anspruch 5,
**dadurch gekennzeichnet, dass** das Injektionsgerät (1) mindestens einen Längssteg (52) besitzt, der parallel zur Längsmittelachse (50) das Injektionsgeräts (1) verläuft, wobei der Längssteg (52) die Rasterhöhung und die Längsführung für den Rastarm (66) bildet.

7. Inj ektionsgerät nach einem der Ansprüche 1 bis 6,
**dadurch gekennzeichnet, dass** die Drehbewegung des Zustellteils (20) beim Einstellen der auszupressenden Menge an Injektionsflüssigkeit über eine erste Gewindeverbindung (25) die axiale Bewegung des Zustellteils (20) bewirkt, wobei das Zustellteil (20) um einen ersten Stellweg (a) in distaler Richtung verschoben wird.

8. Injektionsgerät nach Anspruch 7,
**dadurch gekennzeichnet, dass** die erste Gewindeverbindung (25) ein Innengewinde (65) des Zustellteils (20) umfasst, das mit einem Außengewinde (69) eines drehfest mit dem Gehäuse (2) verbundenen Dosierkolbens (22) zusammenwirkt.

9. Injektionsgerät nach Anspruch 7 oder 8,
**dadurch gekennzeichnet, dass** das Bedienelement (6) mehrteilig ausgebildet ist und einen Betätigungsknopf (8) und eine Stellhülse (7) besitzt, wobei der Betätigungsknopf (8) über einen Mitnehmer (13) mit dem Zustellteil (20) verbunden ist, und wobei die Stellhülse (7) fest mit einem Dosierorgan (16) verbunden ist.

10. Injektionsgerät nach Anspruch 9,
**dadurch gekennzeichnet, dass** der Betätigungsknopf (8) über eine Kupplung (14) mit der Stellhülse (7) verbunden ist, wobei die Kupplung (14) in einer ersten, distalen Position (71) des Betätigungsknopfs (8) eine drehfeste Verbindung zwischen dem Mitnehmer (13) und der Stellhülse (7) herstellt und in einer zweiten, proximalen Position (72) des Betätigungsknopfs (8) eine Drehung der Stellhülse (7) gegenüber dem Mitnehmer (13) zulässt.

11. Injektionsgerät nach Anspruch 9 oder 10,
**dadurch gekennzeichnet, dass** das Dosierorgan (16) über eine zweite Gewindeverbindung (18) mit dem Gehäuse (2) verbunden ist, wobei die Drehbewegung des Dosierorgans (16) eine Bewegung des Dosierorgans (16) und des Bedienelements (6) in distaler Richtung um einen zweiten Stellweg (b) bewirkt, wobei der zweite Stellweg (b) größer als der erste Stellweg (a) ist.

12. Injektionsgerät nach einem der Ansprüche 9 bis 11,
**dadurch gekennzeichnet, dass** das Injektionsgerät (1) einen Schieber (19) besitzt, der ein Gewinde einer dritten Gewindeverbindung (21) trägt, wobei die dritte Gewindeverbindung (21) bei einer Drehbewegung des Schiebers (19) eine Bewegung des Schiebers (19) in distaler Richtung der Längsmittelachse (50) um einen dritten Stellweg (c) bewirkt, der dem ersten Stellweg (a) entspricht.

13. Injektionsgerät nach Anspruch 12,
**dadurch gekennzeichnet, dass** der Schieber (19) drehfest mit dem Dosierorgan (16) und über die dritte Gewindeverbindung (21) mit dem Gehäuse (2) verbunden ist.

14. Injektionsgerät nach Anspruch 12 oder 13,
**dadurch gekennzeichnet, dass** der Schieber (19) einen Mitnahmeabsatz (62) besitzt, der mit einem Mitnahmeabsatz (63) des Zustellteils (20) zusammenwirkt und der eine axiale Bewegung des Schiebers (19) in proximaler Richtung auf das Zustellteil (20) überträgt.

## Claims

1. Injection device having a housing (2), in which a receiving area (5) for a container with injection liquid is formed, having an operating element (6) which can be rotated to set an amount of injection liquid to be expressed from the container and which moves upon rotation in the direction of a longitudinal middle axis (50) of the injection device (1) in the distal direction of the injection device (1) relative to the housing (2), wherein the operating element (6) is displaced to express injection liquid from the container in the direction of the longitudinal middle axis (50) in the proximal direction of the injection device (1), wherein the injection device (1) has a blocking part (20) which is connected rotationally securely to the operating element (6) upon adjustment of the amount of injection liquid to be expressed and is displaced in the direction of the longitudinal middle axis (50) in the distal direction and which, upon expression of injection liquid from the container, is rotationally securely connected to the housing (2) and displaced in the direction of the longitudinal middle axis (50) in the proximal direction,
**characterised in that** the injection device (1) has a blocking contour (55) extending spirally around the longitudinal middle axis (50), which blocking contour (55) cooperates with the blocking part (20) and which blocks a movement of the blocking part (20) in the proximal direction, wherein the blocking contour (55) has on at least one point an interruption (53) which enables a movement of the blocking part (20) in the direction of the longitudinal middle axis (50) in the proximal direction.

2. Injection device according to claim 1,
**characterised in that** the operating element (6), upon adjustment of the amount of injection liquid to be expressed, can be moved into at least one injection position (73), in which an admissible amount of injection liquid is set, and into at least one blocking position (74), in which an inadmissible amount of injection liquid is set, wherein the blocking part (20) lies against the blocking contour (55) in the blocking position (74) and is arranged on the distal side of the interruption (53) of the blocking contour (55) in the injection position (73).

3. Injection device according to claim 2,
**characterised in that** the injection device (1) has a detent means (26) which latches in the injection position (73) and which acts between the blocking part (20) and the housing (2).

4. Injection device according to claim 3,
**characterised in that** the blocking part (20) has at least one detent arm (66) which lies against the blocking contour (55), and the injection device (1) has, in the peripheral direction adjacent to the interruption (53) of the blocking contour (55), at least one raised detent area which forms the detent means (26) with the detent arm (66).

5. Injection device according to claim 4,
**characterised in that** the injection device has a longitudinal guide which connects the blocking part (20) upon movement thereof in the proximal direction rotationally securely to the housing (2).

6. Injection device according to claim 5,
**characterised in that** the injection device (1) has at least one longitudinal web (52) which extends parallel to the longitudinal middle axis (50) of the injection device (1), wherein the longitudinal web (52) forms the raised detent area and the longitudinal guide for the detent arm (66).

7. Injection device according to one of claims 1 to 6,
**characterised in that** the rotation movement of the blocking part (20), upon setting of the amount of injection liquid to be expressed, causes via a first threaded connection (25) the axial movement of the blocking part (20), wherein the blocking part (20) is displaced by a first displacement path (a) in the distal direction.

8. Injection device according to claim 7,
**characterised in that** the first threaded connection (25) comprises an internal thread (65) of the blocking part (20) which cooperates with an external thread (69) of a metering piston element (22) connected rotationally securely to the housing (2).

9. Injection device according to claim 7 or 8,
**characterised in that** the operating element (6) is formed in multiple parts and has an activating button (8) and an adjustment sleeve (7), wherein the activating button (8) is connected via an entrainment element (13) to the blocking part (20), and wherein the adjustment sleeve (7) is fixedly connected to a metering element (16).

10. Injection device according to claim 9,
**characterised in that** the activating button (8) is connected via a coupling (14) to the adjustment sleeve (7), wherein the coupling (14) produces, in a first, distal position (71) of the activating button (8), a rotationally secure connection between the entrainment element (13) and the adjustment sleeve (7), and, in a second, proximal position (72) of the activating button (8), enables a rotation of the adjustment sleeve (7) relative to the entrainment element (13).

11. Injection device according to claim 9 or 10,
**characterised in that** the metering element (16) is connected, via a second threaded connection (18), to the housing (2), wherein the rotation movement of the metering element (16) causes a movement of the metering element (16) and the operating element (6) in the distal direction by a second displacement path (b), wherein the second displacement path (b) is larger than the first displacement path (a).

12. Injection device according to one of claims 9 to 11,
**characterised in that** the injection device (1) has a slider element (19) which carries a thread of a third threaded connection (21), wherein the third threaded connection (21), upon a rotation movement of the slider element (19), causes a movement of the slider element (19) in the distal direction of the longitudinal middle axis (50) by a third displacement path (c) which corresponds to the first displacement path (a).

13. Injection device according to claim 12,
**characterised in that** the slider element (19) is rotationally securely connected to the metering element (16) and via the third threaded connection (21) to the housing (2).

14. Injection device according to claim 12 or 13,
**characterised in that** the slider element (19) has an entrainment section (62) which cooperates with an entrainment section (63) of the blocking part (20) and which transmits an axial movement of the slider element (19) in the proximal direction to the blocking part (20).

## Revendications

1. Dispositif d'injection avec un boîtier (2) dans lequel est formé un logement (5) pour un récipient avec un liquide d'injection, avec un élément de manoeuvre (6) qui est apte à tourner pour régler une quantité de liquide d'injection à expulser du récipient et qui, lors de sa rotation, est déplacé par rapport au boîtier (2) dans le sens distal du dispositif d'injection, dans le sens d'un axe longitudinal médian (50) du dispositif d'injection (1), étant précisé que l'élément de manoeuvre (6), pour l'expulsion de liquide d'injection du récipient, est déplacé dans le sens proximal du dispositif d'injection (1), dans le sens de l'axe longitudinal médian (50), étant précisé que le dispositif d'injection (1) comporte un élément de positionnement (20) qui, lors du réglage de la quantité de liquide d'injection à expulser, est relié fixe en rotation à l'élément de manoeuvre (6) et est déplacé dans le sens distal, dans le sens de l'axe longitudinal médian (50) et qui, lors de l'expulsion de liquide d'injection du récipient, est relié fixe en rotation au boîtier (2) et est déplacé dans le sens proximal, dans le sens de l'axe longitudinal médian (50),
**caractérisé en ce que** le dispositif d'injection (1) comporte un contour de blocage (55) qui s'étend en spirale autour de l'axe longitudinal médian (50), qui coopère avec l'élément de positionnement (20) et qui empêche un déplacement de ce dernier dans le sens proximal, étant précisé que le contour de blocage (55) comporte à au moins un endroit une interruption (53) qui autorise un déplacement dudit élément de positionnement (20) dans le sens proximal, dans le sens de l'axe longitudinal médian (50).

2. Dispositif d'injection selon la revendication 1,
**caractérisé en ce que** l'élément de manoeuvre (6), lors du réglage de la quantité de liquide d'injection à expulser, est apte à être amené dans au moins une position d'injection (73) dans laquelle une quantité autorisée de liquide d'injection est réglée, et dans au moins une position de blocage (74) dans laquelle une quantité non autorisée de liquide d'injection est réglée, étant précisé que l'élément de positionnement (20) est appliqué, dans la position de blocage (74), contre le contour de blocage (55) et est disposé, dans la position d'injection (73), sur le côté distal de l'interruption (53) du contour de blocage (55).

3. Dispositif d'injection selon la revendication 2,
**caractérisé en ce que** le dispositif d'injection (1) comporte un dispositif de verrouillage (26) qui se verrouille dans la position d'injection (73) et qui agit entre l'élément de positionnement (20) et le boîtier (2).

4. Dispositif d'injection selon la revendication 3,
**caractérisé en ce que** l'élément de positionnement (20) comporte au moins un bras de verrouillage (66) qui est appliqué contre le contour de blocage (55), et **en ce que** le dispositif d'injection (1) comporte, près de l'interruption (53) du contour de blocage (55) dans le sens circonférentiel, au moins une saillie de verrouillage qui forme avec le bras de verrouillage (66) le dispositif de verrouillage (26).

5. Dispositif d'injection selon la revendication 4,
**caractérisé en ce que** le dispositif d'injection comporte un guide longitudinal qui relie l'élément de positionnement (20), lors du déplacement de celui-ci dans le sens proximal, fixe en rotation au boîtier (2).

6. Dispositif d'injection selon la revendication 5,
**caractérisé en ce que** le dispositif d'injection (1) comporte au moins une nervure longitudinale (52) qui est parallèle à l'axe longitudinal médian (50) dudit dispositif d'injection (1), étant précisé que la nervure longitudinale (52) forme la saillie de verrouillage et le guide longitudinal pour le bras de verrouillage (66).

7. Dispositif d'injection selon l'une des revendications 1 à 6,
**caractérisé en ce que** le mouvement de rotation de l'élément de positionnement (20) lors du réglage de la quantité de liquide d'injection à expulser provoque par l'intermédiaire d'une première liaison à filetage (25) le déplacement axial de l'élément de positionnement (20), étant précisé que l'élément de positionnement (20) est déplacé sur une première course de réglage (a), dans le sens distal.

8. Dispositif d'injection selon la revendication 7,
**caractérisé en ce que** la première liaison à filetage (25) comprend un filetage intérieur (65) de l'élément de positionnement (20), qui coopère avec un filetage extérieur (69) d'un piston de dosage (22) relié fixe en rotation au boîtier (2).

9. Dispositif d'injection selon la revendication 7 ou 8,
**caractérisé en ce que** l'élément de manoeuvre (6) est en plusieurs parties et comporte une tête d'actionnement (8) et un manchon de réglage (7), étant précisé que la tête d'actionnement (8) est reliée par l'intermédiaire d'un organe d'entraînement (13) à l'élément de positionnement (20), et que le manchon de réglage (7) est relié de manière fixe à un organe de dosage (16).

10. Dispositif d'injection selon la revendication 9,
**caractérisé en ce que** la tête d'actionnement (8) est reliée par l'intermédiaire d'un accouplement (14) au manchon de réglage (7), l'accouplement (14) réalisant dans une première position, distale (71) de la tête d'actionnement (8) une liaison fixe en rotation entre l'organe d'entraînement (13) et le manchon de réglage (7), et autorisant dans une deuxième position, proximale (72) de la tête d'actionnement (8), une rotation du manchon de réglage (7) par rapport à l'organe d'entraînement (13).

11. Dispositif d'injection selon la revendication 9 ou 10,
**caractérisé en ce que** l'organe de dosage (16) est relié par l'intermédiaire d'une deuxième liaison à filetage (18) au boîtier (2), étant précisé que le mouvement de rotation de l'organe de dosage (16) provoque un déplacement de l'organe de dosage (16) et de l'élément de manoeuvre (6) dans le sens distal sur une deuxième course de réglage (b), ladite deuxième course de réglage (b) étant plus grande que la première course de réglage (a).

12. Dispositif d'injection selon l'une des revendications 9 à 11,
**caractérisé en ce que** le dispositif d'injection (1) comporte un coulisseau (19) qui porte un filetage d'une troisième liaison à filetage (21), étant précisé que la troisième liaison à filetage (21) provoque, lors d'un mouvement de rotation du coulisseau (19), un déplacement dudit coulisseau (19) dans le sens distal de l'axe longitudinal médian (50) sur une troisième course de réglage (c) qui correspond à la première course de réglage (a).

13. Dispositif d'injection selon la revendication 12,
**caractérisé en ce que** le coulisseau (19) est relié fixe en rotation à l'organe de dosage (16) et par l'intermédiaire de la troisième liaison à filetage (21) au boîtier (2).

14. Dispositif d'injection selon la revendication 12 ou 13,
**caractérisé en ce que** le coulisseau (19) comporte un épaulement d'entraînement (62) qui coopère avec un épaulement d'entraînement (63) de l'élément de positionnement (20), et qui transmet un déplacement axial du coulisseau (19) dans le sens proximal à l'élément de positionnement (20).
